# EUROPEAN PATENT SPECIFICATION

(11) **EP 0 991 423 B1**
(45) Date of publication and mention of the grant of the patent: **12.09.2001**
(21) Application number: 98930578.4
(22) Date of filing: 23.06.1998
(51) Int. Cl.: A61K 39/395

(54) **TOPICAL TREATMENT OF PSORIASIS USING NEUTRALIZING ANTIBODIES TO IL-8**
LOKALE BEHANDLUNG VON PSORIASIS UNTER VERWENDUNG NEUTRALISIERENDER ANTIKÖRPER GEGEN IL-8
TRAITEMENT TOPIQUE DU PSORIASIS METTANT EN OEUVRE DES ANTICORPS APTES A NEUTRALISER IL-8

(30) Priority: 23.06.1997 CN 97112184
(43) Date of publication of application: 12.04.2000
(73) Proprietor: Yes Biotech Laboratories Ltd., Missisauga, Ontario L5S 1V6 (CA)
(72) Inventor: YE, George, Q., W., Mississauga, Ontario L5V 1N8 (CA)
(74) Representative: Perry, Robert Edward
(86) International application number: CA9800604
(87) International publication number: WO9858671

(56) References cited:
- WO-A-91/16928
- WO-A-98/19706
- Y. JI ET AL.: "Flow cytometry analysis of the neutralization effect of anti-IL-8 mcabs on IL-8-activated human granulocytes." SHIH YEN SHENG WU HSUEH PAO (J. EXP. BIOL.), vol. 28, no. 3, September 1995, pages 257-261, XP002080143 China
- M. KUWAHARA ET AL.: "IL-8 production and chemotaxis in psoriatic fibroblast." NIPPON HIFUKA GAKKAI ZASSHI (JPN. J. DERMATOL.), vol. 105, no. 4, 1995, pages 567-573, XP002080144 Japan
- C. SHAO: "Psoriatic research in China." INTERNATIONAL JOURNAL OF DERMATOLOGY, vol. 31, no. 12, December 1992, pages 840-844, XP002080145 Philadelphia, PA, USA
- B. NICKOLOFF ET AL.: "Aberrant production of interleukin-8 and thrombospondin-1 by psoriatic keratinocytes mediates angiogenesis." AMERICAN JOURNAL OF PATHOLOGY, vol. 144, no. 4, April 1994, pages 820-828, XP002080146 Hagerstown, MD, USA
- A. TUSCHIL ET AL.: "Interleukin-8 stimulates calcium transients and promotes epidermal cell proliferation." THE JOURNAL OF INVESTIGATIVE DERMATOLOGY, vol. 99, no. 3, September 1992, pages 294-298, XP002080147 Baltimore, MD, USA

## Description

### FIELD OF THE INVENTION

The invention relates to pharmaceutical compositions for topical application of mAb to treat psoriasis and other inflammatory skin conditions.

### BACKGROUND OF THE INVENTION

Psoriasis is a common, noncontagious, chronic inflammatory disease of unknown cause. It is a worldwide disease and it's prevalence in the general population is nearly 3% for the people of the Faroe Islands and Denmark. Over 5 million people in the United States are afflicted with this disease (2% of the population).

It most commonly appears as sharply circumscribed salmon pink patches covered with silvery white scales. Diagnosis is usually made by observation and examination of the skin. There are different types of psoriasis that display certain characteristics. Each of these types can range from mild to severe. However, psoriasis is variable and one type can change into another type or several types can exist at the same time. The National Psoriasis Foundation describes the types as follows:
(1) *Plaque Psoriasis:* raised, inflamed lesions that are covered in white scale. Most common types that is also called psoriasis vulgaris. Locations: anywhere, but usually on scalp, elbows, knees, trunk.
*(2) Guttate Psoriasis:* small, drop-like dots with some scale. Location: trunk, legs, arms.
*(3) Inverse Psoriasis:* smooth inflamed lesions, no scale. Location: skin folds, armpit, groin.
*(4) Erthrodermic Psoriasis:* severe sloughing of the skin with redness. Location: anywhere on body.
*(5) Psoriatic Arthritis:* swelling and inflammation of joints can result in 10% of psoriasis patients. Location: knees, hips, elbows, spine, hands and feet.
*(6) Scalp Psoriasis:* is usually plaque type. Affect 50% of psoriasis patients.
*(7) Nail Psoriasis:* pitting, discolouration, and loss of fingernails and toenails. Usually inflammation of skin around the nail.

It has been reported that a combination of genetic, environmental, and immunological factors contribute to the disease. It is believed that a person is predisposed to developing psoriasis, but there appears to be no pattern of inheritance. Only one in three people reports a family history of this disease, whereas others show no incidence of psoriasis in family. There may be important triggers (superantigens such as bacteria, virus, and fungus; vaccinations, intramuscular injection, certain drugs, stress, and injury to the skin; Koebner phenomenon, etc.) that initiate the development of psoriasis in those that are predisposed to developing it. Then as a result of these triggers the immune system causes excessive skin cell reproduction.

In normal skin growth, skin cells produced in the basal cell layer move up through the epidermis to the outermost layer, the stratum comeum. This process from cell birth to cell death takes about 28-30 days. When skin is damaged this cycle is much faster. Although there is no wound at the site of psoriatic lesions, skin cells called keratinocytes act in a regenerative manner. New skin cells are produced in 2-4 days, thus making it very difficult shed old cells at a adequate rate. The elevated scaly lesions are a result of the buildup of cells. The white scale is dead skin cells and the redness is a result of an increase in blood flow to areas of high cell division.

Psoriasis is characterized by (1) extreme epidermal hyperproliferation (excessive growth associated with incomplete and accelerated differentiation) (2) noticeable inflammation of epidermis and dermis at local sites with development of neutrophil microabscess and enhanced induction of cycling T lymphocytes. Thus, the cause of psoriasis was initially thought to involve one of the mediators of hyperproliferation However research began to focus on the immune system after by chance it was discovered that cyclosporine immunosuppressive effects significantly improved psoriasis in patients. Thus it is now viewed as an autoimmune disease. Recently, there has been more elucidation about the pathogenesis of psoriasis. Today there are three main theories of psoriasis origin and development. (1) T-lymphocytes are activated in psoriatic lesions by cytokines that are released from epidermal keratinocytes. (2) Antigen dependent T-cell activation causes the release of cytokines that activate epidermal keratinocytes. (3) Autoimmune reactions of CD8+ "killer" T-lymphocytes with epidermal keratinocytes trigger epidermal activation.

Psoriasis does not affect overall health and is not life threatening, but people do die from complications associated with this disease. The physical and especially the emotional effects of psoriasis can be painful. This disease can cause disfigurements which physically limit, thus affecting job and leisure activities. This causes frustration, embarrassment, fear and depression for psoriasis sufferers, especially with severe types. Psoriasis is persistent and unpredictable in its course, thus no single treatment works for everyone. As a result, there are a variety of treatments available that can be used alone or in combination. These treatments may diminish symptoms transiently but they are not curative. Very often they are aesthetically unpleasant, expensive, time consuming and have side effects that are unhealthy. For the most part, present treatment is unsatisfactory.

In general, mild forms of psoriasis are treated by topical applications of glucocorticoids eg. Corticaine. Keratolytic agents such as sulfur or salicylic acid, are useful adjuvants. Side-effects are mild. Moderate forms of psoriasis are usually treated with anthralin/dithranol or tar preparations eg. Pentrax. Side-effects are mild-moderate. Severe cases of psoriasis or mild to moderate forms that do not respond to conventional therapy may require treatment with systemic medication. Side-effects are usually severe.

Some of the current therapies of psoriasis are as follows:
1. Phototherapy:
   (a) Narrow ban ultraviolet B phototherapy (UVB): burning and carcinogenesis.
   (b) Psoralen with ultraviolet A (PUVA): long term problem of carcinogenesis and short term problems of nausea, phototoxicity, and pruritus
   (c) Photodynamic therapy: limitations include photosensitivity and tissue destruction.
2. Drugs approved for other uses:
   (a) Zidovudine (Retrovir): used to slow AIDS. Side effects involved a decrease in RBC and WBC counts.
   (b) Histamine₂ Receptor Antagonists: used to treat stomach ulcers, i.e. ranitidine (Zantac) and cimetidine (Tagament). Side effects involved an initially worsening of symptoms.
   (c) Antithyroid Thioureylenes: used for hyperthryroidism, i.e. propylthiouracil and methimazole (Tapazole). Side-effects: hypothyroidism, but decreased with a topical formulation
   (d) Capsaicin (Zostrix 0.025% cream): is approved for pain relief in rheumatoid arthritis, osteoarthritis, and neuralgia. The major side effect is stinging.
3. New drugs developed for psoriasis:
   (a) Acitretin (Neotegison/Neotigason): a second-generation monoaromatic retinoid. This drug is teratogenic. Related retinoid Etretinate (Tegison/ Tigason) shows similar effects.
   (b) Fumaric acid therapy: side effects include abdominal disturbances, lymphopenia, flushing, and mild change of hepatic and renal function. In 85% of patients, long term therapy causes lymphopenia.
   (c) Vitamin D derivatives: 1,25 dihydroxyvitamin D₃ (1,25-(OH)₂D₃) shows hypercalciuria in systemic and topical applications. A synthetic 1,24-dihydroxyvitamin D₃ analogue, i.e. Calcipotriene ointment (Dovonex ointment) diminishes hypercalciuria side-effects but results in face and intertriginous irritation. Tacalcitol, also shows face irritation.
   (d) Tazarotene (Tazorac): is an acetylenic retinoid molecule. Topical application showed dose-related irritation.
4. Immune therapy:
   (a)Cyclosporine (Sandimmune): is approved for use in organ transplantation. Some side effects are potentially toxic and are as follows: headaches, gastrointestinal disturbances, hypertrichosis, paresthesias, and gingival hyperplasia. It is extremely important that nephrotoxicity be carefully monitored with this drug. Side-effects increase with length of time the drug is administered, so it is not an acceptable long-term therapy for patients. A new formulation called Neoral (approved for organ transplantation) may reduce toxicity, but further studies are needed.
   (b)DAB₃₈₉IL-2: a cytotoxin that selectively attacks IL-2 receptors on cells and destroys them. Side effects include: flu-like symptoms, pruritus, and transient transaminase elevation.
   (c)Tacrolimus (Prolaf): is a macrolide antibiotic used to treat allograft rejection in liver transplant patients. Side-effects are similar to Cyclosporine.
   (d)CTLA41g: is an experimental agent that blocks the second signal in T-cell activation. Side-effects are unknown. Clinical trials are in progress.
   (e)Anti-CD4 Monoclonal Antibody: side effects include chills and fever. More in depth toxicity studies are needed.
   (f)T-cell receptor peptide vaccines: Vβ3 and Vβ13.1 T-cells are targeted. Clinical trials in progress to determine toxicity
   (g)Other immunologic agents: include TNF-alpha inhibitors and antisense oligonucleotides. Side-effects unknown.

Monoclonal antibody (mAb) preparations may be effective in fighting malignancy, infection, and immune disorders. A monoclonal antibody is directed against and binds to a single epitope on an antigenic molecule. Characteristics such as homogeneous high binding affinity and specificity make them suitable for developing therapeutics. However, mAb preparations for the most part have been administered using systemic drug delivery methods.

Topical treatments are preferred for treating psoriasis and other skin diseases because there are less side-effects. A concerted effort to develop a topical preparation containing antibodies for treating psoriasis has not been undertaken. This is because it has been accepted that a sufficient level of antibodies cannot be absorbed through the skin to combat psoriasis.

It is unknown exactly which biological factors play a role in the manifestation of the disease. This has made it difficult to develop a topical treatment. With a topical treatment, lower levels of antibodies reach the target site. A topical treatment therefore requires the use of an antibody or other active ingredients which can neutralize a biological factor which is directly linked to the manifestation of the disease.

We have found that interleukin-8 (IL-8) or neutrophil-activating protein (NAP-1) plays a significant role in the manifestation of psoriasis and other inflammatory skin conditions. It was not previously known that antibodies or other agents that neutralize IL-8 are effective in the treatment of psoriasis and other inflammatory skin conditions.

There is therefore a need for a topical treatment for treating psoriasis that is effective in neutralizing biological factors that are directly involved in the manifestation of the disease. There is a specific need for a topical treatment for psoriasis that contains antibodies for neutralizing IL-8.

### SUMMARY OF THE INVENTION

The present invention provides a pharmaceutical composition for treating a human subject for psoriasis or other inflammatory skin conditions. The composition comprises an agent that diminishes the effect of psoriasis or other inflammatory skin conditions together with a pharmaceutically acceptable carrier. The present invention provides a method of treating psoriasis or other inflammatory skin conditions through topical administration of a pharmaceutical composition that diminishes the effect of these conditions.

According to one aspect of the present invention, a pharmaceutical composition for topical administration to a patient to treat an inflammatory skin condition is provided. The composition comprises an antibody that diminishes the effect of the inflammatory skin condition. The composition also includes a pharmaceutically acceptable carrier.

According to another aspect of the present invention, a pharmaceutical composition for topical administration to a patient to treat psoriasis is provided. The pharmaceutical composition comprises an antibody that neutralizes interleukin-8.

According to another aspect of the present invention, a pharmaceutical composition for topical administration to a patient to treat psoriasis is provided. The pharmaceutical composition comprises at least one of the following antibodies:
- I8-60
- I8-S2
- 3C6
and a pharmaceutically acceptable carrier.

According to another aspect of the present invention a method of treating psoriasis or other inflammatory skin conditions is provided. The method comprises the step of applying topically a pharmaceutical composition comprising an antibody that neutralizes interleukin-8 and a pharmaceutically acceptable carrier.

According to yet another aspect of the present invention a method of treating psoriasis is provided. The method comprises the step of applying topically a pharmaceutical composition comprising an antibody is effective in diminishing the effects of psoriasis and a pharmaceutically acceptable carrier.

According to yet another aspect of the present invention, use is made of a pharmaceutical composition comprising an antibody that neutralizes interleukin-8 for topically treating psoriasis or other inflammatory skin conditions.

### DESCRIPTION OF THE FIGURES

Figure 1 is a table showing the isotypes of the IL-8 monoclonal antibodies that were identified with Mouse typer sub-isotyping kit;
Figure 2 is a table indicating that monoclonal antibodies 18-S2, 18-60, and 3C6 recognize different epitopes of IL-8 molecule;
Figure 3 is a table setting out the reagents used to prepare a base cream;
Figure 4 is a table summarizing effects of a topical composition containing monoclonal antibodies on psoriasis patients;
Figure 5 is a table summarizing effects of a topical composition containing polyclonal antibodies on psoriasis patients; and
Figure 6 is a table summarizing effects of a topical composition containing polyclonal antibodies on eczema patients.

### DETAILED DESCRIPTION OF THE INVENTION

The monoclonal antibodies outlined in this invention are obtained according to processes that are known per se. General hybridoma techniques are well known, however in certain cases specific problems may require changes to known techniques. There is no certainty that the required hybridoma will be formed and produce specific antibodies, but the degree of success will depend on the completion of the following steps:
(a) Mice were immunized with purified recombinant human interleukin-8 (IL-8, monocyte-derived, 72 a.a. form). The immunization schedule and the IL-8 concentration ("immunogen") should be sufficient to produce satisfactory serum titers of antibodies. Three immunizations with approx. 200 µL of antigen solution every 3-4 weeks by subcutaneous (s.c) and intraperitoneal (i.p.) injection have been found to be effective.
(b) Using well known experimental techniques the spleen cells of the immunized mice were removed 3-4 days after last ("booster") immunization and suspended in an appropriate medium.
(c) The suspended spleen cells are fused with mouse myeloma cells of a suitable cell line with a suitable fusion promoter, preferably polyethylene glycol (PEG) having a molecular weight from 1000 to 4000 . However, other fusion promoters known in the art may be used. Preferably, spleen cells are fused with myeloma cells in a 5:1 ratio.
   Any appropriate mouse myeloma cell line may be used but it is preferred that myeloma cells that do not survive in a selective culture medium containing hypoxanthine, aminopterin and thymidine (HAT) medium be used, such as those that lack enzyme hypoxanthine-guanine-phosphoribosyl transferase (HGPRT) or the enzyme thymidine kinase (TK). Especially preferred are myeloma cells and cell lines that do not survive in HAT medium and do not by itself secrete any antibody, for example the cell lines X63-Ag8.653 and Sp2/0-Ag14.
   After fusion, the cells were cultured in selective HAT medium, which supports the growth of hybridoma cells, not the growth of unfused myeloma cells. Only fused cells continue to grow because they have from the myeloma cells the ability to grow in vitro, and from the spleen cells parent the ability to survive in selective medium.
   Hybridoma cells must be grown in suitable culture media, for example RPMI 1640 medium or Dulbecco's Modified Eagle's Medium. This media is supplemented with 10-15% fetal bovine serum. At the beginning of cell growth "feeder cells" may be added, for example spleen cells, bone marrow, normal mouse peritoneal exudate cells or "hybridoma growth factors".
(d) As soon as the medium has started to turn acidic (yellow) and the cell colonies are visible, a small amount of the cell culture supernatant should be removed to be tested for the presence of the desired antibodies, for example antibody to IL-8.
(e) Wells that test positive for antibody through the screening assay are selected and cloned as soon as possible using well known experimental techniques, for example limiting dilution (easiest) in order to ensure their monoclonality.
(f) The mAb's isotypes were determined using well known methods, for example "dipstick" assay/dot blot or ELISA.
(g) Antibodies were tested for their ability to specifically neutralize human IL-8 activity. Three hybridoma cell lines produced murine antibodies with high neutralizing ability and were deposited at the ATCC, under the Budapest Treaty Deposit Procedure, on May 14 1998, under accession numbers CRL-12528, CRL-12527 AND CRL-12529 for the cell lines designated 18-60 (IL-8-60), 18-S2 (IL-8-S2), and 3C6 (IL-8-3C6), respectively.
(h) 18-60, 18-S2, and 3C6 which bind to different antigenic determinants of IL-8 can be utilized in a "cocktail" for immunotherapy of psoriasis and other inflammatory skin conditions. It is suggested to administer topically to patients suffering from psoriasis and other inflammatory skin conditions, a combination of said antibody together with a pharmaceutically acceptable carrier. Preliminary clinical trials have demonstrated that such a topical composition is effective.
(i) To ensure a good stock of hybridoma cells and the antibody it secretes, it is necessary to grow up the cells after repeated clonings. This can be done by production of ascites fluid or bulk tissue culture. For ascites production the preferred hybridoma is injected into mice, which will grow and cause ascitic fluid containing mAb to form in the abdominal cavity. After a suitable length of time mouse ascites can be collected using well known experimental techniques and may provide up to 10 mg/mL of antibody. For bulk culture, the hybridoma clones are cultured in vitro in a suitable medium using static cultures, roller cultures or bioreactors. After a suitable length of time the supernatant from a standard flask can be collected and may provide between 10 to 50 µg/mL.
(j) The bulk antibodies should be purified using well known experimental techniques to remove all major contaminants, for example affinity chromotography.
(k) A polyclonal antibody may also be used quite satisfactorily as an alternative to the monoclonal antibody "cocktail" for immunotherapy of psoriasis and other inflammatory skin conditions. Polyclonal antibody was prepared by injection of chicken with purified recombinant human interleukin-8 using standard immunization protocols. After a suitable period of time eggs were collected and the chicken yolk IgY was purified. It is suggested to administer topically to patients suffering from psoriasis and other inflammatory skin conditions, a combination of said polyclonal antibody together with a pharmaceutically acceptable carrier. Preliminary clinical trials have demonstrated that such a topical composition is effective.

The following examples illustrate the preferred embodiments of the invention without limiting the scope thereof.

### EXAMPLE 1

### PRODUCTION OF MOUSE ANTI-HUMAN IL-8 MONOCLONAL ANTIBODIES

### 1.1 Immunogen:

Purified recombinant human interleukin-8 (rhlL-8) derived originally from human monocyte was obtained from Pepro Tech, USA. It consists of 72 amino acids, has a molecular weight of 8.5 kDa, purity > 98% by N-terminal assay and SDS-PAGE silver staining, showed strong chemotactic activity to human neutrophils by chemotaxis assay. 1.2 Immunization:

Female BALB/c mice (Charles River Laboratories, Inc., Canada) were immunized with rhlL-8. The immunization procedure was as follows: 200 µL of antigen (20µg rhlL-8/200µL PBS) added to 200µL Freund's Complete Adjuvant to make 400µL antigen emulsified solution. Day 1: this solution was injected subcutaneously (s.c.) into mice at multiple sites on back. Day 27: 400µL antigen solution (20 µg rhIL-8/400µL PBS) was added with 400µL Freund's Incomplete Adjuvant, and mice immunized by intraperitoneal injection (i.p.). Day 59: same as day 27. Day 91: same as day 27. Day 152: immunize mice by i.p. injection with 20 µg rhIL-8/450 µL PBS antigen solution. Day 155: the spleens of the mice were removed and prepared for cell fusion.

### 1.3 Cell fusion:

Mouse SP2/0-Ag 14 myeloma cells (ATCC, CRL 1581) which don't secret heavy or light chain of immunoglobulins were used. It is resistant to 8-azaguanine and fails to survive in HAT medium. SP2/0-Ag14 is widely used as fusion partner to prepare mAb secreting hybridoma.

SP2/0-Ag14 myeloma cells in logarithmic phase were washed with serum-free RPMI 1640 medium twice.

Spleen cell suspension prepared under sterile conditions were washed with serum-free RPMI 1640 medium twice.

Spleen cells and SP2/0-Ag14 cells were mixed in a 5:1 ratio, then centrifuged at 1500 RPM for 7 minutes and supernatant removed. Slowly, 1 mL of 50% PEG4000 (MW:3000-4000) was added (GIBCO BRL, USA), taking 1 minute. The mixture was let to sit for 1.5 min. 5 mL of serum free RPMI 1640 medium was added slowly, taking 2.5 min. The mixture was let to sit for 5 min. The mixture was centrifuged at 1000 RPM for 5 minutes and supernatant removed. Cells re-suspended in regular RPMI 1640 medium containing 15% FBS (GIBCO BRL).

The above cell suspension was distributed 100 µL (2 drops/well) to the 96 well plates (100 µL, well). Plates were placed in a CO₂ incubator (37°C), then HAT culture media was exchanged every 3 days. At day 10, HAT culture media was exchanged for HT media.

After 14 days of incubation, supernatant from wells with growing colonies are screened for their binding capacity to IL-8 with ELISA and anti-lL-8 positive clones were selected.

### 1.4 Cloning of hybridoma:

In limited dilution method, the diluted cell suspension (3-10 cells/mL) was added 2 drops/well to 96 well plate. The plate was then incubated in a CO₂ incubator (37°C). During incubation, every well was exchanged with 1/3 fresh culture RPMI 1640 culture media every 3-4 days. Ten days later, the second screen and cloning were carried out. Clones whose mean cloning rate is <66.7 and mean antibody positive rate is 100% were deemed monoclonals after three successive clonings. There were 9 clones that were deemed monoclonals specific for human IL-8,

### EXAMPLE 2

### CHARACTERIZATION OF MONOCLONAL ANTIBODIES

### 2.1 Immunoglobulin subtypes:

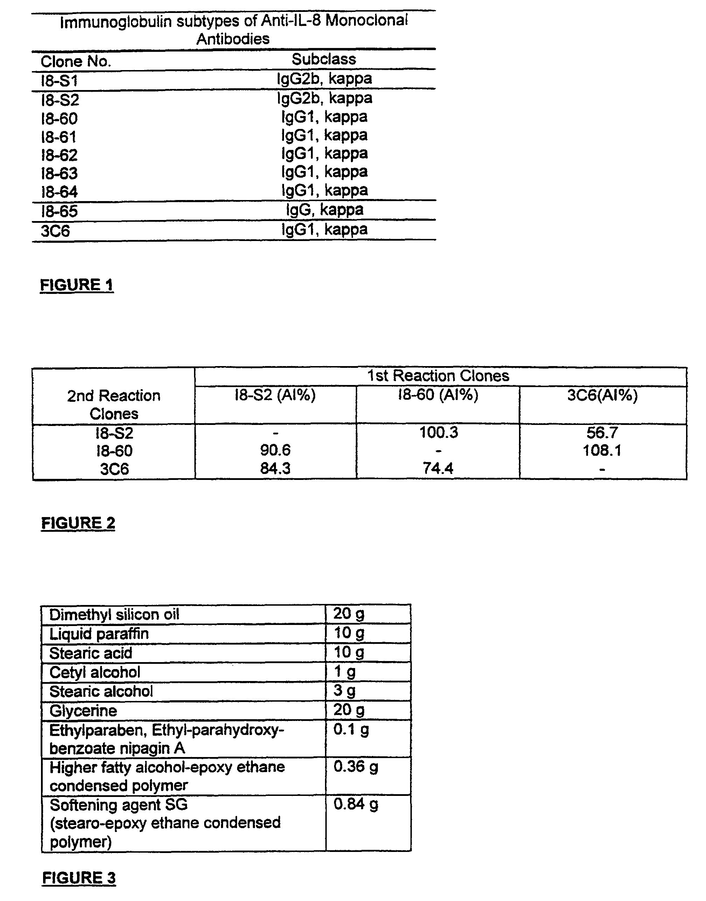

The isotypes of the IL-8 mAbs were identified with the Mouse typer sub-isotyping kit (BioRad, USA). The results are summarized in Figure 1.

### 2.2 Specificity:

All IL-8 mAbs were tested for cross-reaction to various cytokines and chemotactic factors by ELISA. Results showed that those IL-8 mAbs exhibited no cross-reaction with IL-1β, IL-7, IL-16, EGF, M-CSF, GM-CSF, MCAF, MCP-3, TGF-β1, TNF-α and BSA and were specifically reactive to IL-8.

### EXAMPLE 3

### MONOCLONAL ANTIBODY NEUTRALIZING TESTS

The following anti-IL-8 mAbs showed a strong neutralization effect on IL-8:
(a) 18-60 and 3C6: purified antibodies were used in a neutrophil chemotaxis assay. rhIL-8 at 1 µg/mL was incubated with different concentrations of purified 18-60 or 3C6 at 37 deg.C for 45 minutes, then diluted to a final concentration of 50 ng/mL, an optimal dose of rhIL-8 for eliciting neutrophil responses. 26 µg/mL of monoclonal antibodies neutralized 50% of neutrophil chemotactic response to rh IL-8 with 100% neutralization at 80 µg/mL.
(b) 18-S2: through flow cytometry analysis it demonstrated IL-8 could activate human granulocytes and induce elevation of [Ca²⁺]. Neutralizing effect can thus be determined by blocking (Ca²⁺]ᵢ elevation by anti-IL-8 antibodies. Tests for changes of Ca++ concentration in cells with flow cytometry showed that 18-S2 mAb displayed a high neutralizing effect on human granulocytes activated by IL-8.

### EXAMPLE 4

### EPITOPE RECOGNITION OF MONOCLONAL ANTIBODIES WITH NEUTRALIZING ACTIVITY

Recombinant IL-8 at a concentration of 0.02 µg/mL were coated on a 96 well microplate. Using ELISA Additivity Test [Friguet, B. et al. (1983) J. Immunol. Methods, 60:351-358.] index (AI)=[(2OD₁₊₂ )/(OD₁ + OD₂) -1] x 100% was determined. Using this method, an Al>50% indicates that the two antibody clones recognize different epitopes. Results from Figure 2 indicate that mAbs 18-S2, 18-60, and 3C6 recognize different epitopes of IL-8 molecule.

### EXAMPLE 5

### BULK PRODUCTION & PURIFICATION MONOCLONAL ANTIBODIES WITH NEUTRALIZING ACTIVITY

To produce larger quantities of mAbs 18-60, 18-S2, 3C6 the hybridomas were grown in mice.
Day 1: Balb/c mice were injected intraperitoneally with 0.5 mL of Pristane (2,6,10,14-tetramethylpentadecane). Day 7: the hybridoma cells were washed with PBS and 1 x 10⁶ cells were injected into each mouse using i.p. route. After two weeks the ascites fluid was removed using well known experimental techniques.

Recombinant Protein G Agarose (GIBCO BRL, USA) was used to purify IgG antibody from cell culture supernatant or ascites. Binding Buffer (Sodium Phosphate, pH 7.0/0.15M Sodium Chloride) and Eluting Buffer (0.1M Glycine Hydrochloride, pH 2.6) were used for purification.

For every batch of mAbs purified by protein G affinity chromatography the specificity and binding affinity were tested by ELISA method. IL-8 was coated at 0.1 µg/well and the mAb titers at their end point were found to be 0.1 ng/mL (18-60), 0.1 ng - 1 ng/mL (18-S2), and 100 ng/mL (3C6). Purified and quality controlled mAbs were filtered by 0.22µm Sterile Millex-GS filter for sterilization (Millipore, Canada), then lyophilized and stored at -20°C.

### EXAMPLE 6

### PRODUCTION OF CHICKEN ANTI-HUMAN IL-8 POLYCLONAL ANTIBODIES

### 6.1 Immunogen:

Purified recombinant human IL-8.

### 6.2 Immunization:

Shaver Browns laying hen was immunized with recombinant human IL-8. The immunization procedure was as follows: 200 µg IL-8 dissolved in PBS was emulsified with an equal volume of Complete Freund's Adjuvant and a total of 600 µL was injected subcutaneously (s.c) and intramuscularly (i.m.) in various combinations in 4 sites on each breast. Day 17, 29, 42, 56, 118: the hen was boosted with 100 µg of IL-8 emulsified with an equal volume of Freund's Incomplete Adjuvant Day 187: the hen was boosted with 200 µg of IL-8 emulsified with an equal volume of Freund's Incomplete Adjuvant. The hen eggs were collected and the egg yolk IgY was purified by Gallus Immunotech, Canada.
6.3 Specificity: The polyclonal antibody exhibits no detectable cross-reactivity with human serum albumin, and other cytokines tested. .
6.4 Neutralizing Activity: The purified IgY from egg yolks showed potent activity of neutralizing IL-8. Total chicken IgY fraction at 50 µg/mL showed 60% inhibition of 10 ng/mL lL-8 induced IL-8 RB/293 cell migration.
6.5 Product Form: Chicken IgY in phosphate buffered saline, pH 7.3 no preservatives. Stored at -20°C.

### EXAMPLE 7

### PHARMACEUTICAL PREPARATION FOR TOPICAL ADMINISTRATION

### 7.1 Preparation of base cream:

The reagents from figure 3 were weighed and placed in an open stainless steel tank (2000 mL vol.) successively.

The stainless steel tank was placed into a thermostat water bath and heated to 80°C which took approximately 10 minutes. The liquid is thoroughly mixed then emulsifying and homongenating equipment was placed into the open stainless steel tank, the mixture was stirred for 20 minutes at 3500 rpm until fully emulsified. The temperature of the thermostat water bath was cooled naturally to 30-37°C, until the mixture became a semi-solid cream. The mixture is being continually stirred.

### 7.2 Preparation of liquid antibody mixture no.1:

MAbs I8-S2, 3C6, and I8-60 are prepared in accordance with Example 5. For 1000 gm of base cream, 45 mg of total antibody was required, for example 15 mg (clone 18-S2), 15 mg (clone 3C6), and 15 mg (clone 18-60).

Add 4 mL of distilled water to antibody mixture per 100 gm of base prepared, for example 40 mL of distilled water was added to 45 mg antibody mixture to reconstitute to a final concentration of 1.125 mg/mL.

### 7.3 Preparation of liquid antibody mixture no.2:

Polyclonal Chicken Anti-human IL-8 is prepared in accordance with Example 6.. For 1000 gm of base cream, 450 mg of polyclonal antibody was required. A higher mg/gm of cream was needed for polyclonal preparation because about less than 10% specific antibody to IL-8 was contained in whole IgY.

Add 4 mL of distilled water to antibody mixture per 100 gm of base prepared, for example 40 mL of distilled water was added to 450 mg antibody mixture to reconstitute to a final concentration of 11.25 mg/mL.

### 7.4 Preparation of topical composition (monoclonal)

While the base cream is being stirred using emulsifying and homongenating equipment, the liquid antibody mixture no.1 prepared in accordance with Example 7.2 is dropped to base cream prepared in accordance with Example 7.1 using a pasteur aspirating tube. After the antibody mixtureis added, the total mixture is stirred for 10 more minutes. The topical composition is packaged and stored at 4°C.

### 7.5 Preparation of topical composition (polyclonal)

While the base cream is being stirred using emulsifying and homongenating equipment, the liquid polyclonal antibody mixture no.2 is prepared in accordance with Example 7.3 is dropped to base cream prepared in accordance with Example 7.1 using a pasteur aspirating tube. After antibody mixture is added, the total mixture is stirred for 10 more minutes. The topical composition is packaged and stored at 4°C.

### EXAMPLE 8

### TREATMENT OF HUMAN PSORIATIC SKIN USING TOPICAL COMPOSITION (MONOCLONAL)

### 8.1 Materials and Clinical Protocol:

The topical composition was prepared in accordance with Example 7.4. The composition was applied to 29 psoriasis patients (23 plaque, 4 erythrodermic, and 2 arthritic). All patients received approximately 0.2g cream/cm² of lesion area. The cream was applied twice a day for 4 weeks.

Evaluation of effects were divided in 4 grades. These are cured, obvious effective, effective, non-effect.
cured: plaque diminished completely, pruritus disappeared
obvious effect: ≥ 60% plaque diminished, pruritus slightened (softened)
effective: 20% ~ 60% plaque diminished, pruritus slightened (softened).
non-effect less than 20% plaque diminished or exacerbation of psoriasis. Pruritus not softened or deteriorated.

### 8.2 Results of trials:

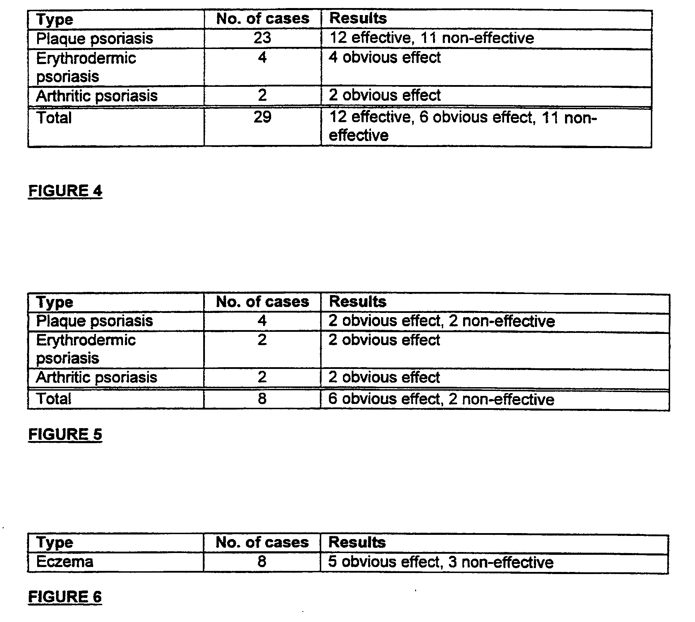

A summary of the effects of the topical composition on the 29 psoriasis patients are shown in figure 4.

The topical composition showed an obvious effect for erythrodermic psoriasis and arthritic psoriasis and may be effective for plaque psoriasis to some degree. No visible side-effects were observed.

This method of treating psoriasis is external, convenient and easy to administer, and shows effectiveness in a short period of time.

### EXAMPLE 9

### TREATMENT OF HUMAN PSORIATIC SKIN USING TOPICAL COMPOSITION (POLYCLONAL)

### 9.1 Materials and Clinical Protocol:

The topical composition was prepared in accordance with Example 7.5. The composition was applied to 8 psoriasis patients (4 plaque, 2 erythrodermic, and 2 arthritic) All patients received approximately 0.2 g cream/cm² of lesion area. The cream was applied twice a day for 4 weeks.

Evaluation of effects were divided in 4 grades. These are cured, obvious effective, effective, non-effect.
cured: plaque diminished completely, pruritus disappeared
obvious effect ≥ 60% plaque diminished, pruritus slightened (softened)
effective: 20% - 60% plaque diminished, pruritus slightened (softened).
non-effect less than 20% plaque diminished or exacerbation of psoriasis. Pruritus not softened or deteriorated.

### 9.2 Results of trials:

A summary of the effects of the topical composition on the 8 psoriasis patients are shown in figure 5.

The topical composition showed an obvious effect for erythromermic psoriasis and arthritic psoriasis and may be effective for plaque psoriasis to some degree. No visible side-effects were observed.

This method of treating psoriasis is external, convenient and easy to administer, and shows effectiveness in a short period of time

### EXAMPLE 10

### TREATMENT OF HUMAN ECZEMIC SKIN USING TOPICAL COMPOSITION (POLYCLONAL)

### 10.1 Materials and Clinical Protocol:

The topical composition was prepared in accordance with Example 7.5. The composition was applied to 8 eczema patients. All patients received approximately 0.2 g cream/cm² on lesion area. The cream was applied twice a day for 4 weeks.

Evaluation of effects were divided in 4 grade. These are cured, obvious effective, effective, non-effect.
cured: plaque diminished completely, pruritus disappeared
obvious effect: ≥ 60% plaque diminished, pruritus slightened (softened)
effective: 20% ~ 60% plaque diminished, pruritus slightened (softened).
non-effect: less than 20% plaque diminished or exacerbation of psoriasis. Pruritus not softened or deteriorated.

### 10.2 Results of trials:

A summary of the effects of the topical composition on the 8 eczema patients are shown in figure 6.

The topical composition showed an obvious effect in 63% of eczema patients. No visible side-effects were observed.

This method of treating eczema is external, convenient, and easy to administer. It shows effectiveness in a short period of time.

The mechanism by which anti-lL-8 antibody is absorbed into psoriatic lesions in a sufficient amount to neutralize IL-8 and be therapeutically effective is unclear. Possibly reasons include:
(1) high enough concentrations of antibodies can be maintained at the epidermal surface for a long period, thus permitting slow penetration of therapeutically effective dose.
(2) the defective permeability barrier in psoriasis may allow for greater penetration of an effective dose of antibodies into the epidermis.

The advantages of the proposed treatment over the present treatment are as follows:
(1) Topical application would minimize the toxic side effects that are often associated with systemic drug delivery because the treatment is applied locally.
(2) Antibodies are unique in that they are specific, homogeneous, and can be produced in vitro at infinitum.
(3) Antibodies to IL-8 specifically neutralizes IL-8 in psoriatic lesions, not anything else.

To produce a more effective cream, other neutralizing agents may be introduced into the topical composition. The neutralizing agent may be a IL-8 receptor blocking agent, for example a peptide that binds to IL-8 receptor site or antibodies to IL-8 receptor (IL-8R) or soluble IL-8 receptors.

Although the invention has been described with preferred embodiments, it is to be understood that modifications may be resorted to as will be apparent to those skilled in the art. Such modifications and variations are to be considered within the purview and scope of the present invention.

### Literature References

1. Krueger, GG and M. Duvic. (1994) Epidemiology of psoriasis: Clinical Issues. *J Invest Dermatol* 102: 14S-18S.
2. The National Psoriasis Foundation-USA
3. Boehncke, W.H. et al. (1996) Pulling the trigger on psoriasis. *Nature* 379: 777.
4. Bruch-Gerharz, D. et al. (1996) A proinflammatory activity of interleukin-8 in human Skin: expression of the inducible nitric oxide syntase in psoriatic lesion and cultured keratinocytes. *J.Exp. Med* 184: 2007-2012.
5. Baggiolini, M. et al. (1994) Interleukin-8 and related chemotactic cytokines-CXC and CC chemokines. *Advances in Immunology. Academic Press*, *Inc.* 55: 97-179.
6. Guzzo, C. (1997) The recent advances in the treatment of psoriasis. *Advances in Clinical Research.* 15(1): 59-68.
7. Kemeny, L et al. (1994) Role of interleukin-8 receptor in skin. *Int. Arch. Allergy Immunol.* 104: 317-322.
8. Gillitzer R. et al. (1991) Upper keratinocytes of psoriatic skin lesion express high levels of NAP-1/IL-8 mRNA in situ. *J Invest. Dermatol.* 97: 73-79.
9. Sticherling M et al: (1991) Localization of neutrophil -activating peptide-1/interleukin-8 immunoreactivity in normal and psoriatic skin. *J Invest. Dermatol.* 96:26-30.
10. Van Damme, J. (1994) Interleukin-8 and related chemotactic cytokines. In: *The Cytokine Handbook* (Thompson, A eds.), Academic Press: Toronto, pp. 185-208.
11. Schroder, J-M and Christophers, E. (1986) Identification of C5a des arg and an anionic neutrophil-activating peptide (ANAP) in psoriatic scales. *J. Invest. Dermatol.* 87: 53-58.
12. Van Damme J et al. (1988) A novel NH₂-terminal sequence characterized human monokine possessing neutrophil chemotactic, skin-reactive, and granulocytosis-promoting activity. *J Exp. Med.* 167:1364-1376.
13. Larsen CG. et al. (1989). The neutrophil activating-protein (NAP-1) is chemotactic for T lymphocytes. *Science.* 243: 1464-1466.
14. Michel G et al. (1992) Interleukin-8 receptor-mediated chemotaxis of normal human epidermal cells. *FEBS lett.* 305:241-243.
15. Tuschil A, et al. (1992) Interleukin-8 simulates calcium transients and promotes epidermal cell proliferation. *J Invest. Dermatol.* 99: 294-298.
16. Nickoloff, B.J et al. (1994) Aberrant production of Interleukin-8 and thrombospondin-1 by psoriatic keratinocytes mediates angiogenesis. *American Journal of Pathology* 144(4): 820-828
17. Huber, AR et al. (1991) Regulation of transedothelial migration by endogenous interleukin-8. Science. 253: 1278-1280.
18. Kulke, R. et al. (1988) The CXCR receptor is overexpressed in psoriatic epidermis. J *Invest Dermatol.* 110: 90-94.
19. Ghadially, R et al. (1996) Stratum comeum structure and function correlates with phenotype in psoriasis. *J. Inves. Dermatol.* 107: 558-564.
20. Fartasch, M. (1997) Epidermal barrier disorders of the skin. *Microsc. Res. Tech.* 38: 361-372.
21. Motta, S et al. (1994) Interlamellar lipid differences between normal and psoriatic stratum corneum. *Acta. Derm. Venenerol (Stockh),* Suppl. 186:131-132.
22. Ji, Y-Y and G. Ye. (1995) Flow cytometry analysis of the neutralization effect of anti-IL-8 mcAbs on IL-8 activated human granulocytes. *Acta Biologiae Experimentalis Sinica* 28(3): 257-261.

## Claims

1. A pharmaceutical composition for topical administration to treat an inflammatory skin condition, comprising an antibody that neutralizes interleukin-8.

2. A composition according to claim 1, wherein the antibody is a monoclonal antibody.

3. A composition according to claim 1, wherein the antibody is a polyclonal antibody.

4. A composition according to claim 1, wherein the antibody is an antibody fragment.

5. A composition according to claim 1, comprising at least one of antibodies 18-60 (ATCC accession No. CRL-12528), 18-S2 (ATCC accession No. CRL-12527) and 3C6 (ATCC accession No. CRL-12529).

6. A composition according to any preceding claim, which comprises a pharmaceutically acceptable carrier selected from a neutral sterile cream, gel, jelly, ointment, aerosol, patch and powder.

7. A composition according to any preceding claim, wherein the acceptable carrier is a cream.

8. A composition according to any preceding claim, wherein the condition is psoriasis or eczema.

9. Use of an antibody that neutralizes interleukin-8, for the manufacture of a composition as defined in any of claims 1 to 7, for treating an inflammatory skin condition.

10. Use according to claim 9, wherein the condition is psoriasis.

## Patentansprüche

1. Pharmazeutische Zusammensetzung für die topische Verabreichung, um einen Hautentzündungszustand zu behandeln, welche einen Antikörper umfasst, der Interleukin-8 neutralisiert.

2. Zusammensetzung nach Anspruch 1, in welcher der Antikörper ein monoklonaler Antikörper ist.

3. Zusammensetzung nach Anspruch 1, in welcher der Antikörper ein polyklonaler Antikörper ist.

4. Zusammensetzung nach Anspruch 1, in welcher der Antikörper ein Antikörper-Fragment ist.

5. Zusammensetzung nach Anspruch 1, umfassend mindestens einen der Antikörper 18-60 (ATCC-Zugangsnr. CRL-12528), 18-S2 (ATCC-Zugangsnr. CRL-12527) und 3C6 (ATCC-Zugangsnr. CRL-12529).

6. Zusammensetzung nach irgendeinem vorangehenden Anspruch, die einen pharmazeutisch annehmbaren Träger umfasst, der aus einer neutralen sterilen Creme, Salbe, einem neutralen sterilen Gel, Gallert, Aerosol, Pflaster und Pulver ausgewählt ist.

7. Zusammensetzung nach irgendeinem vorangehenden Anspruch, in welcher der annehmbare Träger eine Creme ist.

8. Zusammensetzung nach irgendeinem vorangehenden Anspruch, in welcher der Zustand Psoriasis oder Ekzem ist.

9. Verwendung eines Antikörpers, der Interleukin-8 neutralisiert, für die Herstellung einer Zusammensetzung, die in irgendeinem der Ansprüche 1 bis 7 definiert ist, zur Behandlung eines Hautentzündungszustandes.

10. Verwendung nach Anspruch 9, bei der der Zustand Psoriasis ist.

## Revendications

1. Composition pharmaceutique pour l'administration topique, destinée au traitement d'une affection inflammatoire cutanée, comprenant un anticorps qui neutralise l'interleukine-8.

2. Composition suivant la revendication 1, dans laquelle l'anticorps est un anticorps monoclonal.

3. Composition suivant la revendication 1, dans laquelle l'anticorps est un anticorps polyclonal.

4. Composition suivant la revendication 1, dans laquelle l'anticorps est un fragment d'anticorps.

5. Composition suivant la revendication 1, comprenant au moins un des anticorps 18 - 60 (n° de dépôt ATCC CRL-12528), 18 - S2 (n° de dépôt ATCC CRL-12527) et 3C6 (n° de dépôt ATCC CRL-12529).

6. Composition suivant l'une quelconque des revendications précédentes, qui comprend un support pharmaceutiquement acceptable choisi entre une crème, un gel, une gelée, une pommade, un aérosol, un timbre et une poudre stériles, neutres.

7. Composition suivant l'une quelconque des revendications précédentes, dans laquelle le support acceptable est une crème.

8. Composition suivant l'une quelconque des revendications précédentes, dans laquelle l'affection est le psoriasis ou l'eczéma.

9. Utilisation d'un anticorps qui neutralise l'interleukine-8, pour la production d'une composition répondant à la définition suivant l'une quelconque des revendications 1 à 7, à des fins de traitement d'une affection inflammatoire cutanée.

10. Utilisation suivant la revendication 9, dans laquelle l'affection est le psoriasis.
